# FASCICULE DE BREVET EUROPEEN

(11) **EP 0 920 910 B1**
(45) Date de publication et mention de la délivrance du brevet: **22.01.2003**
(21) Numéro de dépôt: 98402822.5
(22) Date de dépôt: 13.11.1998
(51) Int. Cl.: B01J 27/132, B01J 27/128, B01J 27/125, B01J 23/86, C07C 17/20, C07C 19/08

(54) **Catalyseur mixte de fluoration**
Fluorierungsmischkatalysator
Fluorination mixed catalyst

(30) Priorité: 20.11.1997 FR 9714564
(43) Date de publication de la demande: 09.06.1999
(73) Titulaire: Atofina, 92800 Puteaux (FR)
(72) Inventeur: Bonnet, Philippe, 69007 Lyon (FR); Jorda, Eric, 69005 Lyon (FR); Lacroix, Eric, 69480 Amberieux d'Azergues (FR)

(56) Documents cités:
- EP-A- 0 486 333
- EP-A- 0 609 123
- US-A- 5 616 820
- DATABASE WPI Section Ch, Week 7622 Derwent Publications Ltd., London, GB; Class E16, AN 76-41355X XP002074515 & SU 466 202 A (UNIV TASHK)

## Description

La présente invention a pour objet un catalyseur mixte de fluoration à base de nickel et de chrome. Elle concerne aussi un procédé de fluoration d'hydrocarbures halogénés en phase gazeuse au moyen d'acide fluorhydrique (HF) mettant en oeuvre ledit catalyseur.

La fluoration catalytique d'hydrocarbures halogénés en phase gazeuse par HF est une méthode bien connue d'accès aux hydrocarbures fluorés, et notamment aux hydrofluorocarbures (HFC). Ces derniers sont actuellement utilisés comme substituts des chlorofluorocarbures (CFC) qui sont suspectés de contribuer à l'affaiblissement de la couche d'ozone stratosphérique.

La demande de brevet français FR 2669022 donne ainsi à connaître un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

La teneur pondérale en nickel et chrome mentionnée pour le dit catalyseur est comprise entre 0,5 et 20 % pour chaque métal, le rapport atomique nickel/chrome étant compris entre 0,5 et 5. Ce catalyseur est utilisé pour la préparation du tétrafluoro-1,1,1,2 éthane (également dénommé F134a) à partir du chloro-1-trifluoro--2,2,2 éthane (F133a). Le F134a est un HFC notamment utilisé pour la réfrigération et la congélation, qui ne présente aucun effet néfaste sur la couche d'ozone stratosphérique.

La demande FR 2669022 mentionne un maintien de l'efficacité dans le temps du catalyseur pour une durée allant jusqu'à environ 400 heures de fonctionnement.

Il est cependant très désirable de disposer d'un catalyseur qui, dans les conditions de production industrielle des hydrocarbures fluorés, conserve son activité sur une période de fonctionnement de plusieurs milliers d'heures.

L'augmentation de la durée de vie du catalyseur pour de telles périodes de fonctionnement est d'autant plus souhaitable qu'il ne peut être remédié à la diminution d'activité, lorsqu'elle survient, par un traitement de régénération in situ, mais qu'il est alors nécessaire d'arrêter l'unité de production pour renouveler la charge du catalyseur.

Un catalyseur permettant de diminuer le nombre d'arrêts de l'unité de production et permettant, pour une charge donnée dudit catalyseur, la production d'une plus grande quantité de produit final désiré, est particulièrement avantageux sur le plan de la conduite de l'unité de production, et du coût de revient du produit final désiré.

Un but de l'invention est de proposer un catalyseur de fluoration mixte à base de nickel et de chrome conservant son activité sur une période de fonctionnement de plusieurs milliers d'heures.

Un autre but de l'invention est de proposer un catalyseur permettant d'effectuer des réactions de fluoration à une température plus élevée.

Un autre but de l'invention est de proposer un catalyseur de fluoration dont la charge, dans le réacteur industriel, doit être renouvelée moins fréquemment.

Un autre but de l'invention est de proposer un procédé de fluoration d'hydrocarbures halogénés plus économique.

Un autre but de l'invention est de proposer un procédé de fabrication du tétrafluoro-1,1,1,2 éthane (F134a) présentant un taux de conversion et une sélectivité élevés.

Il a à présent été trouvé que ces buts peuvent être atteints, en totalité ou en partie, au moyen du catalyseur et du procédé de fluoration décrits ci-après.

La présente invention a donc pour objet en premier lieu un catalyseur de fluoration mixte comprenant un ou plusieurs oxydes, halogénures et/ou oxyhalogénures de nickel et de chrome déposés sur un support constitué de fluorure d'aluminium ou d'un mélange de fluorure d'aluminium et d'alumine, caractérisé en ce que le rapport poids de nickel/poids de chrome est compris entre 0,08 et 0,25, de préférence entre 0,1 et 0,2. Ledit catalyseur de fluoration possède une durée de vie qui, de façon surprenante, est très largement améliorée par rapport à celle du catalyseur enseigné par le brevet FR 2669022.

Le catalyseur selon l'invention peut contenir en poids de 0,1 à 6 % de sels de nickel et de 1 à 20 % de sels de chrome, de préférence de 0,35 à 4,5 % de sels de nickel et de 3 à 16 % de sels de chrome, et encore plus préférentiellement, respectivement, de 1 à 2 % et de 6 à 12 %.

Les pourcentages indiqués précédemment sont des pourcentages en poids, exprimés sous la forme du poids équivalent de métal. Sauf mention contraire, tous les pourcentages concernant la composition du catalyseur selon l'invention sont des pourcentages en poids.

Comme sels de chrome et de nickel, on préfère utiliser les chlorures, mais on peut aussi employer d'autres sels tels que, par exemple, les oxalates, formiates, acétates, nitrates et sulfates ou le bichromate de nickel pourvu que ces sels soient solubles dans la quantité d'eau susceptible d'être absorbée par le support.

Le catalyseur selon l'invention peut être préparé de façon connue en soi à partir d'une alumine. Celle-ci peut, dans une première étape, être transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200 et 450°C, de préférence entre 250 et 400°C). Le support est ensuite imprégné à l'aide de solutions aqueuses d'anhydride chromique, de sel de nickel et d'un réducteur du chrome comme le méthanol.

Lorsque l'on utilise l'anhydride chromique (CrO₃) comme précurseur du chrome, on peut réduire ce chrome par tout moyen connu de l'homme de l'art (réducteur chimique, réduction thermique ...), sous réserve que la technique utilisée ne nuise pas aux propriétés du catalyseur et donc à son activité. Le réducteur chimique préféré est le méthanol.

Le catalyseur selon l'invention peut aussi être préparé par imprégnation directe de l'alumine à l'aide des solutions des composés de chrome et de nickel ci-dessus mentionnées. Dans ce cas, la transformation d'au moins une partie (70 % ou plus) de l'alumine en fluorure d'aluminium s'effectue lors de l'étape d'activation du catalyseur, généralement réalisée avant sa mise en oeuvre.

Les alumines utilisables, et définies ci-après, pour la préparation du catalyseur selon la présente invention sont des produits bien connus, disponibles dans le commerce. Elles sont généralement préparées par calcination d'hydrates d'alumine à une température comprise entre 300 et 800°C, et peuvent contenir des teneurs importantes (jusqu'à 1000 ppm) de sodium sans que cela ne nuise aux performances catalytiques.

Avant sa mise en oeuvre, le catalyseur selon l'invention doit être conditionné, c'est-à-dire transformé en constituants actifs et stables (aux conditions réactionnelles) par une opération préalable dite d'activation.

Ce traitement peut être réalisé soit "in situ" (dans le réacteur de fluoration) ou bien dans un appareillage adéquat conçu pour résister aux conditions d'activation. Celle-ci comprend généralement une ou plusieurs des étapes suivantes :
- séchage à basse température (100 à 250°C, de préférence 110 à 200°C) en présence d'air ou d'azote,
- séchage à haute température (250 à 450°C, de préférence 300 à 350°C) sous azote ou sous air,
- fluoration à basse température (180 à 300 °C, de préférence à 200°C environ) au moyen d'un mélange d'acide fluorhydrique et d'azote, en contrôlant la teneur en HF de façon que la température ne dépasse pas 350°C, et
- finition sous courant d'acide fluorhydrique pur ou dilué par de l'azote à une température pouvant aller jusqu'à 450°C.

Pendant cette opération, les précurseurs catalytiques (halogénures de nickel et de chrome, chromate, bichromate de nickel, oxyde de chrome) sont transformés en fluorures et/ou oxyfluorures correspondants, ce qui entraîne un dégagement d'eau et/ou d'acide chlorhydrique.

Cette activation contribue également à accroître la fluoration de l'alumine lorsque l'on a réalisé l'imprégnation sur un support déjà partiellement fluoré, ou lorsque l'on imprègne directement l'alumine, à la fluoration de cette dernière. Dans ce dernier cas, il est nécessaire de contrôler parfaitement la température (la fluoration de l'alumine est très exothermique) si l'on ne veut pas nuire aux caractéristiques physiques du catalyseur; par ailleurs, les quantités d'eau générées sont nettement plus importantes.

L'analyse chimique des éléments (chrome, nickel, fluor, aluminium, oxygène), après activation, permet de vérifier la composition minérale du catalyseur selon l'invention.

La forme activée du catalyseur est, au même titre que la forme non activée, comprise dans le catalyseur objet de la présente invention.

La durée de vie désirable pour le catalyseur selon l'invention est évaluée selon le test décrit ci-après.

### Test de la durée de vie du catalyseur :

### A - Essai de vieillissement du catalyseur.

Dans un réacteur tubulaire de diamètre intérieur de 27 mm en INCONEL 600 (alliage comprenant du nickel, du chrome et du fer dans les proportions respectives de 75 %, 15 % et 8 % en poids), on charge 5 ml de catalyseur activé, selon l'invention. Ce catalyseur est ensuite porté à une température de 490°C en étant maintenu sous un flux N₂/HF (50 %/50 % molaire) de 1 mole de mélange par heure.

Lorsque la température de 490°C est atteinte, le catalyseur est alors soumis à un flux de HF de 1 mole/heure contenant 1 % molaire de O₂, durant 24 heures.

Cet essai de vieillissement accéléré est représentatif des conditions de fonctionnement d'un catalyseur de fluoration durant plusieurs milliers d'heures.

### B - Performance du catalyseur.

La performance du catalyseur est évaluée avant et après essai de vieillissement accéléré de la façon suivante.

Cette performance est testée par l'aptitude à catalyser la fluoration du F133a en F134a par action de HF, et est représentée par le taux de conversion du F133a.

Les conditions opératoires de cette réaction sont les suivantes :
- volume de catalyseur : 5 ml dans un réacteur tubulaire identique à celui utilisé précédemment ;
- température : 350°C
- pression : atmosphérique
- temps de contact : 0,5 s
- rapport molaire HF/F133a : 4
- durée : 24 heures.

### C - Durée de vie du catalyseur.

Celle-ci est donc appréciée par la différence des taux de conversion du F133a enregistrée en présence du catalyseur avant et après essai de vieillissement, conformément aux conditions opératoires décrites précédemment.

Dans les conditions de ce test, on estime qu'une différence de moins de 3 % indique que la durée de vie du catalyseur est, en conditions industrielles, de plusieurs milliers d'heures.

L'invention a également pour objet un procédé de fluoration d'hydrocarbures halogénés en phase gazeuse, au moyen d'acide fluorhydrique, mettant en oeuvre le catalyseur selon l'invention.

Le procédé selon l'invention convient aux hydrocarbures halogénés aussi bien saturés qu'insaturés, et notamment oléfiniques. Il convient particulièrement bien à la fabrication d'hydrocarbures fluorés comprenant de 1 à 3 atomes de carbone et 1 ou plusieurs atomes d'hydrogène. Comme exemples d'hydrocarbures halogénés de départ, on peut mentionner, à titre non limitatif, les composés suivants : CHCl₃, CCl₂=CHCl, CHCl₂-CClF₂, CH₂Cl-CF₃, CH₃-CCl₂-CH₃, CCl₃-CF₂-CH₃, CCl₃-CF₂-CHCl₂, CCl₃-CF₂-CH₂Cl, CHCl₂-CHCl-CH₃, CH₂Cl-CHCl-CH₃, CCl₂=CCl₂, ainsi que CF₃-CH=CHF, CH₂Cl₂,CH₂ClF, CCl₂=CH-CCl₂H, CCl₃-CH = CHCI, CHCl₂-CCl₂F, CHCl-CF₃, CHFCl-CF₃, CH₂Cl₂, CH₂ClF, CHF₂Cl, CHFCl₂.

On préfère mettre en oeuvre le procédé selon l'invention pour la préparation du tétrafluoro-1,1,1,2 éthane (F134a) à partir du chloro-1-trifluoro-2,2,2 éthane (F133a). L'utilisation du catalyseur selon l'invention apporte une plus grande commodité pour la conduite de l'unité industrielle, notamment la possibilité de fixer occasionnellement une température plus élevée. On obtient également dans ce cas une sélectivité en F134a avantageusement élevée.

La température de la réaction de fluoration est en général comprise entre 50 et 500°C, selon les produits de départ et l'hydrocarbure fluoré désiré. On préfère opérer entre 300 et 500°C lorsque l'on souhaite substituer la totalité des atomes de chlore par des atomes de fluor.

Le temps de contact, défini comme étant le rapport "volume du catalyseur/débit total des réactifs" (mesuré dans les conditions de réaction), peut varier dans de larges limites et est généralement compris entre 3 et 100 secondes. En pratique, on préfère travailler à des temps de contact compris entre 5 et 30 secondes, de manière à obtenir à la fois un taux de conversion et une productivité avantageusement élevés.

Le rapport molaire HF/hydrocarbure halogéné de départ varie également dans de larges limites selon notamment la nature de cet hydrocarbure et la stoechiométrie de la réaction. Il est généralement compris entre 1 et 20, de préférence entre 2 et 10.

La pression de fonctionnement est comprise entre 1 et 20 bars absolus (0,1 à 2 MPa), de préférence entre 5 et 16 bars.

Le catalyseur selon l'invention peut fonctionner en lit fixe ou en lit fluidisé. On préfère le lit fixe lorsque la réaction n'est pas exothermique.

Selon une variante préférée du procédé de l'invention, la réaction de fluoration est conduite en présence d'oxygène. On préfère utiliser un rapport "O₂/hydrocarbure halogéné" de départ compris entre 0,001 et 10, de préférence entre 0,5 et 5 (% molaire). L'introduction d'oxygène permet avantageusement de restaurer l'activité du catalyseur lorsque celle-ci diminue occasionnellement en raison du dépôt, sur sa surface, de produits tels que le coke.

Les exemples suivants illustrent l'invention, et ne peuvent en aucun cas être interprétés comme une limitation de celle-ci.

### Exemple 1 :

### A Préparation du catalyseur.

Dans un évaporateur rotatif, on place 300 ml de AlF₃ obtenu par fluoration d'alumine en lit fluidisé vers 300°C à l'aide d'air et d'acide fluorhydrique (concentration volumique de 5 à 10 % de cet acide dans l'air). L'alumine de départ présente les caractéristiques physicochimiques suivantes :
- forme : billes de 1-2 mm de diamètre ;
- surface BET: 223 m²/g
- volume poreux : 1,2 cm³/g (pour les rayons de pores compris entre 40 Å et 63 microns) ;
- teneur en sodium : 900 ppm.

On prépare par ailleurs deux solutions aqueuses séparées :
- une solution d'anhydride chromique (CrO₃) additionnée de chlorure de nickel hexahydraté ;
- une solution méthanolique contenant 3 à 4,5 équivalents molaires de méthanol par mole de chrome.

Le mélange de ces deux solutions est ensuite introduit, à température ambiante sous pression atmosphérique et en 45 minutes environ, sur AlF₃ sous agitation. Les quantités de sels de chrome et de nickel sont ajustées pour obtenir les équivalents molaires désirés en Cr et Ni dans le catalyseur et indiqués dans le tableau suivant.

### B - Actiyation.

Dans un réacteur tubulaire de diamètre intérieur de 27 mm en INCONEL 600, on charge 5 ml du catalyseur précédemment préparé. On procède à un séchage à 200°C sous courant d'azote, pendant 4 heures.

Le catalyseur est ensuite progressivement fluoré par introduction d'un mélange HF/N₂ (50 %/50 % molaire) et en portant (en 5 heures) la température de 200°C à 350°C, cette dernière étant maintenue durant 8 heures. L'étape d'activation est terminée par un traitement avec HF pur à 350°C pendant 10 heures.

Ces catalyseurs sont ensuite soumis au test de durée de vie précédemment défini. Le résultat de ce test est indiqué dans le tableau sous la forme du taux de conversion du 133a avant et après vieillissement accéléré (exprimé en % molaire).

Les taux de conversion et les sélectivités sont également exprimés dans le présent texte en % molaire.

| **Catalyseur** | **% Ni** | **% Cr** | **Rapport Ni/Cr** | **Taux de conversion du 133 a** | |
|---|---|---|---|---|---|
| | | | | *avant vieillissement accéléré* | *après vieillissement accéléré* |
| A | 1 | 6 | 0,17 | 10,2 | 11,3 |
| B | 1 | 9 | 0,11 | 15,6 | 12,7 |
| C | 1,5 | 9 | 0,17 | 9,1 | 10,4 |
| D | 2 | 9 | 0,22 | 13,1 | 10 |
| E | 2 | 12 | 0,17 | 13,5 | 12,3 |

Ces résultats montrent que pour les catalyseurs A à E selon l'invention, la diminution du taux de conversion du F133a après essai de vieillissement est limitée à un maximum de 3 %. Un tel comportement indique pour ces catalyseurs une durée de vie de plusieurs milliers d'heures dans les conditions de fonctionnement d'une unité industrielle de fluoration.

### Exemple 2 : Fluoration du F133a en F134a.

Les performances des catalyseurs de l'exemple 1 ont été testées (après activation) dans les conditions opératoires suivantes :
- volume de catalyseur (en vrac) : 75 ml
- température : 350°C
- pression : atmosphérique
- débit d'acide fluorhydrique : 1,09 moles/h
- débit de F133a : 0,26 moles/h
c'est-à-dire un rapport molaire HF/F133a de 4,2 et un temps de contact de 3,9 secondes dans les conditions réactionnelles.

Les gaz issus de la réaction sont débarrassés des hydracides par lavage à l'eau, puis séchés et analysés par chromotographie en phase vapeur.

On obtient un taux de conversion du F133a de 21 %, une sélectivité de 99 % et une productivité en F134a de 75 g/h/l.

### Exemple 3 :

On répète l'exemple 2, en introduisant également dans le milieu réactionnel 1 % (en mole) de O₂ par rapport au 133a.

Après 1000 heures de fonctionnement, on observe un taux de conversion du F133a supérieur à 20 %, une sélectivité et une productivité en F134a supérieures, respectivement, à 97 % et 60 g/h/l.

### Exemple 4 :

On répète l'exemple 2, en introduisant dans le milieu réactionnel 1,5 % (en mole) de O₂ par rapport au 133a, et en opérant à une température de 420°C et une pression de 15 bars absolus. Le débit des réactifs est le suivant :
- débit d'HF : 2,15 moles/heure
- débit de 133a : 1,08 moles/heure
soit un rapport molaire HF/133a égal à 2 et un temps de contact de 22 secondes.

On observe un taux de conversion du F133a d'environ 22 %, et une sélectivité ainsi qu'une productivité en F134a supérieures, respectivement, à 90 % et 290 g/h/l.

### Exemple comparatif :

On répète l'exemple 1 en préparant un catalyseur Ni/Cr sur support AlF₃ comprenant 6 % de nickel et 6 % de chrome. On obtient un taux de conversion du 133a avant vieillissement de 12,8 % qui chute à 3,2 % après l'essai de vieillissement.

## Revendications

1. Catalyseur de fluoration mixte comprenant un ou plusieurs oxydes, halogénures et/ou oxyhalogénures de nickel et de chrome déposés sur un support constitué de fluorure d'aluminium ou d'un mélange de fluorure d'aluminium et d'alumine, **caractérisé en ce que** le rapport poids de nickel/poids de chrome est compris entre 0,08 et 0,25, de préférence entre 0,1 et 0,2.

2. Catalyseur selon la revendication 1, **caractérisé en ce qu'**il contient en poids de 0,1 à 6 % de sels de nickel et de 1 à 20 % de sels de chrome, de préférence de 0,35 à 4,5 % de sels de nickel et de 3 à 16 % de sels de chrome, et encore plus préférentiellement, respectivement, de 1 à 2 % et de 6 à 12 %.

3. Procédé de fluoration d'hydrocarbures halogénés en phase gazeuse, au moyen d'acide fluorhydrique, mettant en oeuvre le catalyseur selon l'une des revendications 1 ou 2.

4. Procédé selon la revendication 3, **caractérisé en ce que** les hydrocarbures halogénés sont choisis parmi les composés suivants : CHCl₃, CCl₂=CHCl, CHCl₂-CClF₂, CH₂Cl-CF₃, CH₃-CCl₂-CH₃, CCl₃-CF₂-CH₃, CCl₃-CF₂-CHCl₂, CCl₃-CF₂-CH₂Cl, CHCl₂-CHCl-CH₃, CH₂Cl-CHCl-CH₃, CCl₂=CCl₂, CF₃-CH=CHF, CH₂Cl₂, CH₂ClF, CCl₂=CH-CCl₂H, CCl₃-CH = CHCl, CHCl₂-CCl₂F, CHCl₂-CF₃, CHFCI-CF₃, CH₂Cl₂, CH₂ClF, CHF₂Cl, CHFCl₂.

5. Procédé selon l'une des revendications 3 ou 4, **caractérisé en ce que** l'on prépare le tétrafluoro-1,1,1,2 éthane (F134a) à partir du chloro-1-trifluoro-2,2,2 éthane (F133a).

6. Procédé selon l'une des revendications 3 à 5, **caractérisé en ce que** la température est comprise entre 50 et 500°C, de préférence entre 300 et 500°C.

7. Procédé selon l'une des revendications 3 à 6, **caractérisé en ce que** le temps de contact est compris entre 3 et 100 secondes, de préférence entre 5 et 30 secondes.

8. Procédé selon l'une des revendications 3 à 7, **caractérisé en ce que** le rapport molaire "HF/hydrocarbure halogéné de départ" est compris entre 1 et 20, de préférence entre 2 et 10.

9. Procédé selon l'une des revendications 3 à 8, **caractérisé en ce que** la pression de fonctionnement est comprise entre 1 et 20 bars absolus, de préférence entre 5 et 16 bars.

10. Procédé selon l'une des revendications 3 à 9, **caractérisé en ce que** la réaction de fluoration est conduite en présence d'oxygène, avec un rapport "O₂/hydrocarbure halogéné de départ" compris entre 0,001 et 10, de préférence entre 0,5 et 5 % molaire.

## Claims

1. Mixed fluorination catalyst comprising one or more nickel and chromium oxides, halides and/or oxyhalides deposited on a support composed of aluminium fluoride or of a mixture of aluminium fluoride and alumina, **characterized in that** the weight of nickel/weight of chromium ratio is between 0.08 and 0.25, preferably between 0.1 and 0.2.

2. Catalyst according to Claim 1, **characterized in that** it contains, by weight, from 0.1 to 6% of nickel salts and from 1 to 20% of chromium salts, preferably from 0.35 to 4.5% of nickel salts and from 3 to 16% of chromium salts and, more preferably still, from 1 to 2% and from 6 to 12% respectively.

3. Process for the fluorination of halogenated hydrocarbons in the gas phase by means of hydrofluoric acid employing the catalyst according to either of Claims 1 and 2.

4. Process according to Claim 3, **characterized in that** the halogenated hydrocarbons are chosen from the following compounds: CHCl₃, CCl₂=CHCl, CHCl₂-CClF₂, CH₂Cl-CF₃, CH₃-CCl₂-CH₃, CCl₃-CF₂-CH₃, CCl₃-CF₂-CHCl₂, CCl₃-CF₂-CH₂Cl, CHCl₂-CHCl-CH₃, CH₂Cl-CHCl-CH₃, CCl₂=CCl₂, CF₃-CH=CHF, CH₂Cl₂, CH₂ClF, CCl₂=CH-CCl₂H, CCl₃-CH=CHCl, CHCl₂-CCl₂F, CHCl₂-CF₃, CHFCl-CF₃, CH₂Cl₂, CH₂ClF, CHF₂Cl or CHFCl₂.

5. Process according to either of Claims 3 and 4, **characterized in that** 1,1,1,2-tetrafluoroethane (F134a) is prepared from 1-chloro-2,2,2-trifluoroethane (F133a).

6. Process according to one of Claims 3 to 5, **characterized in that** the temperature is between 50 and 500°C, preferably between 300 and 500°C.

7. Process according to one of Claims 3 to 6, **characterized in that** the contact time is between 3 and 100 seconds, preferably between 5 and 30 seconds.

8. Process according to one of Claims 3 to 7, **characterized in that** the "HF/starting halogenated hydrocarbon" molar ratio is between 1 and 20, preferably between 2 and 10.

9. Process according to one of Claims 3 to 8, **characterized in that** the operating pressure is between 1 and 20 bar absolute, preferably between 5 and 16 bar.

10. Process according to one of Claims 3 to 9, **characterized in that** the fluorination reaction is carried out in the presence of oxygen, with an "O₂/starting halogenated hydrocarbon" ratio of between 0.001 and 10, preferably between 0.5 and 5, mol %.

## Patentansprüche

1. Fluorierungsmischkatalysator, umfassend ein oder mehre Oxide, Halogenide und/oder Oxyhalogenide des Nickels und des Chroms, aufgebracht auf einem Träger, der aus Aluminiumfluorid oder einer Mischung aus Aluminiumfluorid und Aluminiumoxid besteht,
**dadurch gekennzeichnet,**
**daß** das Verhältnis Nickelgewicht/Chromgewicht zwischen 0,08 und 0,25, vorzugsweise zwischen 0,1 und 0,2, liegt.

2. Katalysator nach Anspruch 1, **dadurch gekennzeichnet, daß** er gewichtsbezogen 0,1 bis 6 % Nickelsalze und 1 bis 20 % Chromsalze, vorzugsweise 0,35 bis 4,5 % Nickelsalze und 3 bis 16 % Chromsalze, besonders bevorzugt 1 bis 2 % Nickelsalze und 6 bis 12 % Chromsalze, enthält.

3. Verfahren zur Gasphasenfluorierung von halogenierten Kohlenwasserstoffen mit Fluorwasserstoff unter Verwendung des Katalysators nach Anspruch 1 oder 2.

4. Verfahren nach Anspruch 3, **dadurch gekennzeichnet, daß** die halogenierten Kohlenwasserstoffe ausgewählt sind aus den folgenden Verbindungen: CHCl₃, CCl₂=CHCl, CHCl₂-CClF₂, CH₂Cl-CF₃, CH₃-CCl₂-CH₃, CCl₃-CF₂-CH₃, CCl₃-CF₂-CHCl₂, CCl₃-CF₂-CH₂Cl, CHCl₂-CHCl-CH₃, CH₂Cl-CHCl-CH₃, CCl₂=CCl₂, CF₃-CH=CHF, CH₂Cl₂, CH₂ClF, CCl₂=CH-CCl₂H, CCl₃-CH=CHCl, CHCl₂-CCl₂F, CHCl₂-CF₃, CHFCl-CF₃, CH₂Cl₂, CH₂ClF, CHF₂Cl und CHFCl₂.

5. Verfahren nach Anspruch 3 oder 4, **dadurch gekennzeichnet, daß** man 1,1,1,2-Tetrafluorethan (F134a) ausgehend von 1-Chlor-2,2,2-trifluorethan (133a) herstellt.

6. Verfahren nach einem der Ansprüche 3 bis 5, **dadurch gekennzeichnet, daß** die Temperatur zwischen 50 und 500 °C, vorzugsweise zwischen 300 und 500 °C, liegt.

7. Verfahren nach einem der Ansprüche 3 bis 6, **dadurch gekennzeichnet, daß** die Kontaktzeit zwischen 3 und 100 Sekunden, vorzugsweise zwischen 5 und 30 Sekunden, liegt.

8. Verfahren nach einem der Ansprüche 3 bis 7, **dadurch gekennzeichnet, daß** das Molverhältnis "HF/halogenierter Ausgangskohlenwasserstoff" zwischen 1 und 20, vorzugsweise zwischen 2 und 10, liegt.

9. Verfahren nach einem der Ansprüche 3 bis 8, **dadurch gekennzeichnet, daß** der Arbeitsdruck zwischen 1 und 20 bar absolut, vorzugsweise zwischen 5 und 16 bar absolut, liegt.

10. Verfahren nach einem der Ansprüche 3 bis 9, **dadurch gekennzeichnet, daß** die Fluorierungsreaktion in Gegenwart von Sauerstoff mit einem Verhältnis von "O₂/halogenierter Ausgangskohlenwasserstoff" zwischen 0,001 und 10, vorzugsweise zwischen 0,5 und 5 Mol-%, durchgeführt wird.
